Europäisches Patentamt

European Patent Office

Office européen des brevets

(11) Publication number: **0 119 827**
**B1**

# EUROPEAN PATENT SPECIFICATION

(45) Date of publication of patent specification: **06.07.88**

(51) Int. Cl.⁴: **A 41 B 13/02**

(21) Application number: **84301720.3**

(22) Date of filing: **14.03.84**

(54) **Continuous method for elasticizing discrete articles cut from a moving web in a direction perpendicular to web travel.**

(30) Priority: **18.03.83 US 476733**

(43) Date of publication of application:
**26.09.84 Bulletin 84/39**

(45) Publication of the grant of the patent:
**06.07.88 Bulletin 88/27**

(84) Designated Contracting States:
**AT BE CH DE FR IT LI LU NL SE**

(56) References cited:
**EP-A-0 017 770**
**US-A-3 912 565**
**US-A-4 261 782**
**US-A-4 284 454**
**US-A-4 293 367**
**US-A-4 324 245**

(73) Proprietor: **THE PROCTER & GAMBLE COMPANY**
**301 East Sixth Street**
**Cincinnati Ohio 45201 (US)**

(72) Inventor: **Chapman, L. Richard**
**997 Ligorio Avenue**
**Cincinnati Ohio 45218 (US)**
Inventor: **Fanta, Wayne I.**
**2790 Townterrace Drive**
**Cincinnati Ohio 45239 (US)**

(74) Representative: **Gibson, Tony Nicholas et al**
**Procter & Gamble (NTC) Limited Whitley Road**
**Longbenton Newcastle upon Tyne NE12 9TS**
**(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

### BACKGROUND OF THE INVENTION
Field of the Invention

This invention concerns a continuous, high speed method for elasticizing articles such as disposable diapers, incontinent briefs, and the like by securing an elastically contractible element to a moving, interconnected web of said articles, said elastically contractible element causing the discrete articles cut from the web to shirr in a direction substantially perpendicular to the direction of web travel. Said elastically contractible element is preferably applied to said moving web while in a substantially untensioned, molecularly oriented, dimensionally heat unstable condition. The discrete articles cut from the web are thereafter subjected to an external stimulus, such as heat, to cause the elastically contractible element to shrink and become dimensionally heat stable and elastic thereby imparting shirring in a direction substantially perpendicular to the direction of web travel.

Background Art

Infants (and other incontinents) wear disposable diapers to receive and contain urine, feces, and other body fluids. Disposable diapers function both to contain the discharged materials and to isolate these materials from the body of the wearer and from the wearer's surroundings. Modern embodiments of disposable diapers frequently perform these tasks in a manner superior to that of traditional cloth diapers.

Disposable diapers normally comprise three elements: a liquid permeable topsheet designed to be placed next to the wearer's skin; a liquid impermeable backsheet which forms, in use, the outer surface of the diaper; and an absorbent element interposed between the topsheet and the backsheet.

The topsheet is frequently a hydrophobic nonwoven fabric which is readily permeable to fluid. Its hydrophobicity tends to cause the surface in contact with the wearer's skin to be dry and the skin to be protected from fluids absorbed within the absorbent element.

The absorbent element receives and retains fluids which pass through the topsheet. It normally comprises a batt of airlaid wood pulp fibers.

The backsheet functions to contain fluids within the absorbent element thereby protecting the wearer's outer garments and other surfaces from soiling by these fluids. Backsheets are commonly formed of fluid impermeable materials such as polyethylene film.

Disposable diapers having many different basic designs are known in the art. For example, Duncan and Baker in U.S. Patent Re 26,151, issued January 31, 1967, describe and claim a disposable diaper which has achieved wide acceptance and commercial success. Buell, in U.S. Patent 3,860,003, issued January 14, 1975, describes and claims another disposable diaper which, too, has achieved wide acceptance and commercial success. The diaper taught by Buell differs from that taught by Duncan and Baker in many respects, not the least of which is the provision in the Buell diaper of elasticized (or contractible) leg cuffs.

Mesek et al. in U.S. Patent 4,324,245, issued April 13, 1982; Pieniak et al. in U.S. Patent, 4,337,771, issued July 6, 1982; and Mesek et al, in U.S. Patent 4,352,355, issued October 5, 1982 describe disposable diapers having elasticized cuffs and elasticized (or contractible) waistbands.

Strickland and Visscher in U.S. Patent 4,253,461, issued on March 3, 1981, describe and claim another form of disposable diaper, sometimes referred to as an incontinent brief, intended to be worn by adults.

Imparting elasticization to discrete articles has taken various forms. In order to permit economical manufacture of the articles, particularly those which are intended to be disposed of after a single use, it is critical that the attachment method not significantly impact upon either the labor costs associated with manufacturing the article or the production speeds and efficiencies at which said article can be produced. One particularly preferred method for continuously attaching discrete stretched elastic strands to predetermined isolated portions of disposable absorbent products is disclosed in U.S. Patent 4,081,301 issued to Buell on March 28, 1978. The inventive concept disclosed in the Buell patent, in a broad sense, relates to the continuous adherence of discrete lengths of stretched elastic to predetermined portions of a continuously moving web at relatively high operating speeds and thereafter severing the elastic in the unadhered areas to produce elasticized structures having discrete strands of elastic adhered thereto at predetermined locations along their length. In a particularly preferred embodiment, the aforementioned process is utilized to apply discrete elastic legbands to a disposable diaper structure to provide improved containment and fit therein.

An alternative apparatus and method for attaching elastic strips during the manufacture of elastic leg disposable diapers is disclosed in U.S. Patent 4,261,782 issued to Teed on April 14, 1981. According to the process disclosed in the Teed patent, the elastic strips are fed in the direction of web travel and are alternately stretched and relaxed during the attachment process.

Still other approaches to providing shirred, elastic, flexible articles involve the application of an oriented elastic material to the article to be elasticized in an untensioned state and thereafter subjecting the oriented elastic material to heat to cause shrinkage and consequently shirring by restoring the material to its unoriented and elastic state. Exemplary of such processes are those shown in U.S. Patent 3,912,565 issued to Koch et al. on October 14, 1975, U.S. Patent 3,639,917 issued to Althouse on February 8, 1972 and U.S. Patent 3,819,401 issued to Massengale et al. on June 25, 1974.

Koch discloses a method wherein a thin layer of flexible polyurethane material, which is heat shrinkable in the desired direction of article shirring, is disposed in direct contact with at least one side of a flexible sheet material and attached thereto without any significant shrinkage or other distortion of such layer. Subsequently, the layer of polyurethane material is heated to cause shrinkage thereof, whereby the sheet material attached thereto is shirred. The thermoplastic polyurethane resins employed by Koch may be shaped into the desired layer by conventional procedures, as for example by extruding in a molten condition as a continuous stream, followed by rapid quenching of such stream as on a chill roll or in a bath of cool water or other liquid. The layer of polyurethane material thus formed may be collected for subsequent processing at a later stage or, in continuous operations, may be uniaxially stretched directly after its formation. This may be accomplished as by stretching such layer of polyurethane material longitudinally between spaced pairs of differential speed rolls; that is, with one such pair of rolls being rotated at a more rapid speed than the other of such pair of rolls. The uniaxially oriented layer of polyurethane material is preferably heat set by being brought to a temperature slightly above its second order phase transition temperature, permitted a limited relaxation, and then cooled to the temperature of the ambient atmosphere. Once cooled the layer is dimensionally stable. In the example disclosed in the Koch patent, lengths of the heat shrinkable, heat set tape created by the foregoing method are removed from a supply roll and are placed in direct contact with a garment body adjacent to the openings to be elasticized. The attached tape lengths are then heated to shrink the same and cause the portions of the article body to which they are attached to shirr or gather.

Similar approaches utilizing other elastomeric materials are disclosed in the aforementioned patents to Althouse and Massengale et al.

In order to facilitate economical production, and hence disposability, of articles such as disposable diapers, incontinent briefs and the like, it is essential that the means of elasticizing these articles be capable of high speed and great reliability. Since articles such as disposable diapers are typically produced by forming a continuous web of the articles interconnected to one another at their adjacent waistband portions, processes such as those disclosed in the aforementioned U.S. Patent 4,081,301 to Buell have functioned well in the application of stretched elastic bands in a direction generally parallel to the direction of web travel to form elasticized leg cuffs. However, when it is desired to apply an elastic element intended to cause shirring of the finished article in a direction generally perpendicular to the direction of web travel at high speed while the web is moving, none of the processes generally described in the above mentioned prior art patents are particularly appropriate.

Joa U.S. Patent 4284454 discloses an apparatus and method for attachment of elastic bands to a moving web comprised of absorbent articles such as diapers, where the elastic bands are applied to the web in a transverse direction to that of the web movement. In the Joa apparatus, a moving web of airfelt core material is formed into a succession of discrete notched diaper cores, oriented transversely of the direction of core movement, by means of vacuum extraction of the airfelt material from between the cores. A continuous web of impermeable backsheet material provided with adhesive lines extending along the length of the web is applied to the underside of the succession of discrete notched cores. Two strips of elastic ribbon from separate sources are fed transversely of the web, being spaced from each other in the direction of web movement so as to form the respective leg opening elastic bands in an assembled diaper. Predetermined lengths of the ribbon are gripped, tensioned by stretching, held in the stretched state and then cut in an indexing apparatus, The stretched lengths of elastic band are then each transferred from the transversely oriented indexing apparatus to a separate applicator mechanism which comprises an endless conveyor belt orientated in a direction parallel to the direction of web movement. This mechanism includes clamps that maintain the elastic bands in stretched condition and applicator heads that apply the stretched bands to the adhesive coated web of backsheet in the regions thereof exposed by the notching of the diaper cores. The topsheet and upper tissue web components are then applied to the core-backsheet subassembly and are bonded thereto whilst the elastic bands are maintained in stretched condition. Finally the topsheet and backsheet webs are cut to form the individual finished diapers and the elastic bands are released from tension, allowing the side edges of the diapers to become pleated.

The present invention differs from the apparatus and method disclosed in Joa by not requiring any transverse feed of components, all operations being conducted in-line. Furthermore the invention utilises an elastically contractible element, of length equal to the width of the moving web, which is applied to the web so as to extend transversely thereof while the element is in a substantially untensioned, molecularly oriented, heat unstable condition. Subsequent application of heat to the element causes contraction to occur in a direction transverse to that of web movement.

Accordingly, it is an object of the present invention to provide a high speed method for continuously elasticizing articles such as disposable diapers, incontinent briefs, and the like by securing an elastically contractible element to a moving, interconnected web of said articles, said elastically contractible element causing discrete articles cut from the web to shirr in a direction substantially perpendicular to the direction of web travel. Said elastically contractible element is preferably applied to said moving web while in a substantially untensioned, molecularly oriented, heat unstable condition, and is thereafter subjected to an external stimulus, such as heat. The application of heat is preferably done after the discrete articles have been cut from the web and are in a substantially untensioned state to impart shirring.

It is a further object of the present invention to carry out the foregoing process by preparing a web of heat unstable, elastically contractible material having a width which corresponds to the length of the elastically contractible elements to be cut from said web, said web being molecularly oriented in its heat unstable state such that the application of an external stimulus, such as heat, causes shrinkage of said elastically contractible elements in a direction parallel to their length, i.e, parallel to the width dimension of the web from which said elements are cut.

It is a further object of the present invention to apply the external stimulus, such as heat, which causes shrinking along the length of said elastically contractible elements when the articles to which said elements are secured are in a substantially untensioned state.

It is a further object of the present invention to apply said external stimulus to said elastically contractible elements while said articles are folded into a predetermined cross-section to vary the degree of shirring imparted to the article along the length of the elastically contractible elements in a predetermined manner.

Disclosure of the Invention

According to the present invention there is provided a continuous method for elastizing substantially inelastic articles by securing elastically contractible elements to a moving, interconnected web of said articles, said elastically contractible elements causing the discrete articles cut from said web to shirr in a direction substantially perpendicular to the direction of web travel, said method comprising the steps of:

(a) subjecting a web of elastomeric material capable of exhibiting dimensionally heat stable and dimensionally heat unstable states to lateral stretching while said web is at an elevated temperature which is less than its crystalline melting temperature;

(b) allowing said elastomeric web to cool to ambient temperature while in a laterally stretched condition to establish a heat unstable state in the material comprising said web;

(c) feeding said web of elastomeric heat unstable material in a direction substantially parallel to said moving web of interconnected articles;

(d) severing discrete segments of said elastomeric heat unstable material from said elastomeric web in a direction substantially perpendicular to the direction of web travel, whereby each of said discrete segments of elastomeric material has its length oriented parallel to the direction of said lateral stretching and its width extending in a direction substantially parallel to the direction of web travel;

(e) transferring said discrete, severed segments of elastomeric material in a substantially untensioned condition and while their lengths are oriented substantially perpendicular to the direction of web travel to said moving web of interconnected articles at isolated, predetermined locations along the length of said web;

(f) securing said discrete segments of substantially untensioned, heat unstable elastomeric material to said web of interconnected articles at predetermined spaced locations along the length of said segments, as measured in a direction substantially perpendicular to the direction of web travel;

(g) severing each of the interconnected articles from one another, thereby forming a multiplicity of discrete articles, each having at least one segment of substantially untensioned elastomeric material in a heat unstable condition secured thereto; and

(h) heating said discrete articles to an elevated temperature which is less than the crystalline melting temperature of said heat unstable segments of substantially untensioned elastomeric material, thereby causing said elastomeric material to contract in a direction substantially parallel to its overall length, to become heat stable and elastic, and to shirr said articles in a direction substantially perpendicular to said direction of web travel.

In a particularly preferred embodiment, the method of the present invention is employed to produce a disposable diaper including an elastically contractible waistband which, upon return to its molecularly unoriented, heat stable state, imparts a shaped configuration to the waistband portion of the diaper and, at least in part, maintains the diaper in said shaped configuration.

The elastically contractible waistband is itself specially formed and comprises an elastomeric material which has both a dimensionally heat unstable state and a dimensionally heat stable and elastic state (hereinafter referred to, for convenience, simply as "elastomeric material"). "Elastic", as used herein describes a material which can be elongated to a practical extent upon the application of tension and which will substantially return to its original configuration after the tension is released. In the context of the present invention, the heat stable state is dimensionally smaller than the heat unstable state.

The dimensionally heat unstable elastic material can be prepared by techniques such as tentering in a direction perpendicular to the direction of web travel. The web of heat unstable elastomeric material is thereafter continuously fed in the same direction as the web of interconnected diapers, and discrete segments of the heat unstable elastomeric web are severed and applied to each disposable diaper while the elastomeric material is in its heat unstable state. Because the length of each elastomeric waistband element in the diaper corresponds to the width of the elastomeric web from which the elements are cut, the direction of shrinkage of the elastomeric elements upon heating is substantially perpendicular to the direction of web travel.

The disposable diaper is preferably folded into a preselected cross-section, severed from the web to produce a substantially untensioned state and while in that orientation is heated to such an extent as to cause the elastomeric elements to shrink in a direction parallel to their length and assume a heat stable and elastic state. The resulting elastically contractible waistband assumes an essentially permanent set related to the folded configuration, which essentially permanent set tends to cause the disposable diaper to be shaped and to fit more closely about the trunk of the wearer. The resulting waistband also tends to resist waistband rollover.

Brief Description of the Drawings

While the specification concludes with claims particularly and distinctiy claiming the present invention, it is believed the present invention wiil be better understood from the following description in conjunction with the accompanying drawings in which:

Figure 1 is a perspective view of a disposable diaper embodying an elastically contractible waistband shown in a configuration as applied to an infant;

Figure 2 is a partially fragmented plan view of a disposable diaper of the type generally shown in Figures 1 and 11, but in an unfolded, heat unstable configuration;

Figure 3 is an enlarged partial view of the waistband of the diaper of Figure 2 illustrating one preferred embodiment of an elastically contractible waistband in a dimensionally heat stable condition;

Figure 4 is an enlarged partial view of the waistband of the diaper of Figure 2 illustrating another preferred embodiment of an elastically contractible waistband in a dimensionally heat stable condition;

Figure 5 is an end view typical of the portion of the waistbands shown in Figures 3 and 4;

Figure 6 is a simplified schematic representation of an end view of the disposable diaper of Figure 10, taken along view line 6-6, said diaper having its elastomeric waistband material shown in a heat unstable state;

Figure 7 is a simplified schematic end view of the disposable diaper shown in Figure 6 after the elastomeric waistband material has been caused to assume its heat stable and elastic state while in a substantially planar condition;

Figure 8 is a simplified schematic representation of the end of the disposable diaper of Figure 10, said diaper having its elastomeric waistband material shown in a heat unstable state, said diaper having been folded into a C-fold configuration;

Figure 9 is a simplified schematic representation of the disposable diaper shown in Figure 8 after the elastomeric material has been caused to assume its heat stable and elastic state while in a C-fold configuration;

Figure 10 is a partially fragmented plan view of a simple disposable diaper;

Figure 11 is a perspective view of a diaper such as that shown in Figure 1 after it has been returned to a heat stable condition while in a C-fold configuration, said diaper being shown in a condition as applied to an infant;

Figure 12 is a simplified schematic view of a preferred method for forming a continuous roll of heat unstable elastomeric material which will shrink in a direction substantially parallel to the width of the roll upon the application of heat; and

Figure 13 is a simplified schematic view of a preferred method for attaching discrete elastically contractible elements cut from a heat unstable roll of elastomeric material, such as that shown in Figure 12, to a continuously moving web used to form a multiplicity of interconnected elasticized articles which will shirr in a direction substantially perpendicular to the direction of web travel when the articles are cut from the web and subjected to heat in a substantially untensioned state.

Detailed Description of the Invention

For purposes of illustration, the method of the present invention will be described in relation to the continuous fabrication of disposable diaper structures having elasticized waistbands, wherein its benefits are readily apparent. It is of course recognized that the invention may be practiced to advantage in many alternative forms which are defined by the claims appended hereto.

Disposable diapers typically comprise three major elements: a topsheet; a backsheet; and an absorbent element. The topsheet forms the inside of the disposable diaper (i.e., that portion intended to be placed next to the wearer's skin). The backsheet generally forms the exterior surface of the disposable diaper. The absorbent element is interposed between the topsheet and the backsheet.

A disposable diaper is generally designed to be placed between and generally centered between the legs of an infant and secured about the infant by bringing the front portion of the diaper adjacent the front waist area of the infant and the rear portion of the diaper adjacent the rear waist area of the infant and securing the diaper in that position.

Optionally, disposable diapers comprise fastening tapes for securing the diaper about the infant. They also optionally comprise elastic members in the longitudinally extending margins to form an elastically contractible leg cuff or side flap. For example, see U.S. patent 3,860,003 issued to Buell on January 14, 1975.

They also optionally comprise elastic elements in the laterally extending margins to form elastically contractible waistbands.

The waistband of a disposable diaper is that portion of the diaper which is intended to be placed adjacent the wearer's waist. While the waistband can comprise a separate element affixed to the body of the disposable diaper, it more often is an extension of other elements of the disposable diaper such as the backsheet or the topsheet or both the backsheet and the topsheet. Further, the waistband is generally considered to be that portion of the diaper extending from the laterally extending margin of the diaper to about the laterally extending margin of the absorbent element. Disposable diapers are normally constructed so as to have two waistbands: a front and a rear.

In the diaper embodiments herein disclosed for purposes of illustration, the elastically contractible waistband comprises an elastomeric material having both a heat unstable state and a heat stable and elastic state. The elastomeric material is attached to the disposable diaper when in its heat unstable state and is thereafter subjected to heating under conditions hereinafter defined in such a manner that it assumes its heat stable and elastic state.

Massengale et al, in U.S. Patent 3,819,401, issued June 25, 1974 and Koch et al. in U.S. Patent 3,912,565, issued October 14, 1975, teach what appears to be a low speed technique for preparing shirred, or gathered, elastic garments. In this technique, a heat contractible material is affixed to the garment when the heat contractible material is in its elongated and heat unstable state. The entire system is then heated, causing the heat contractible material to contract to its heat stable but elastic state, thereby shirring, or gathering the garment. The present invention differs from Massengale et al. and Koch et al. in that it provides a continuous, high speed method for elasticizing discrete articles cut from a moving interconnected web of said articles in a direction substantially perpendicular to the direction of web travel. This is of critical importance if the cost of said articles must be sufficiently low to permit disposability after a single use.

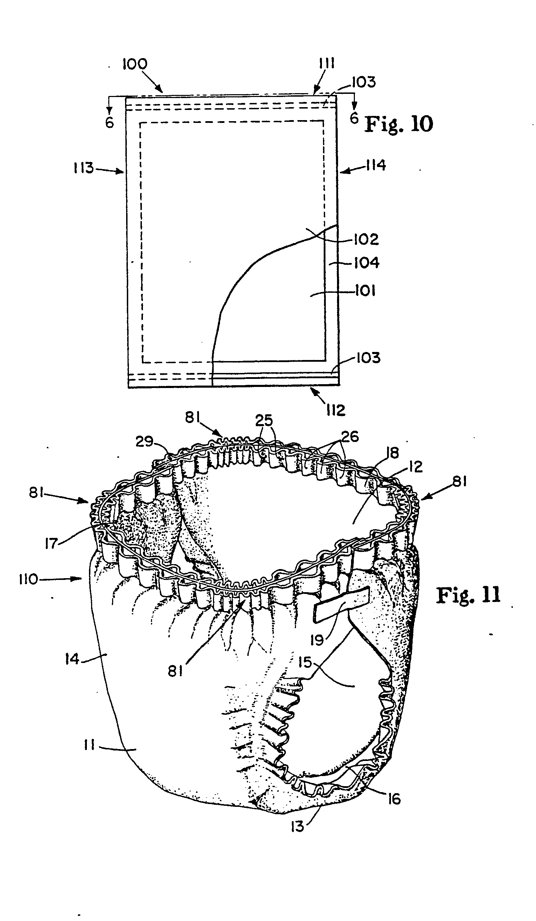

Nearly any prior art disposable diaper, such as those taught by Duncan and Baker or by Buell or by Aziz and Blaney, comprising an absorbent core interposed between a topsheet and a backsheet, can incorporate a strip of an elastomeric material (hereinafter defined in greater specificity) applied laterally across the width of the disposable diaper in one or both of its waist regions. Typically, the absorbent core does not extend fully to the lateral (waist) edges of the topsheet and backsheet which are typically coextensive. The elastomeric material is conveniently interposed between the topsheet and the backsheet in that region between the termination of the absorbent core and the termination of the essentially coextensive topsheet and backsheet. A simple disposable diaper embodiment is shown in partially cut away plan view in Figure 10.

In Figure 10, simple disposable diaper 100 comprises absorbent core 101 interposed between topsheet 102 and backsheet 104. Diaper 100 has longitudinal side margins 113 and 114. It also has lateral waist regions 111 (rear) and 112 (front). Strips of elastomeric material l03 are interposed between topsheet 102 and backsheet 104 and extend laterally across the diaper in that region between the termination of absorbent core 101 and the lateral edges of diaper 10O. Elasto-

meric material strips 103 are affixed to both topsheet 102 and backsheet l04 at spaced locations along their length by securement means known in the art and therefore not illustrated.

It must be noted that reference numerals are used consistently throughout all the figures and that the thicknesses of certain materials in the figures have been exaggerated for clarity.

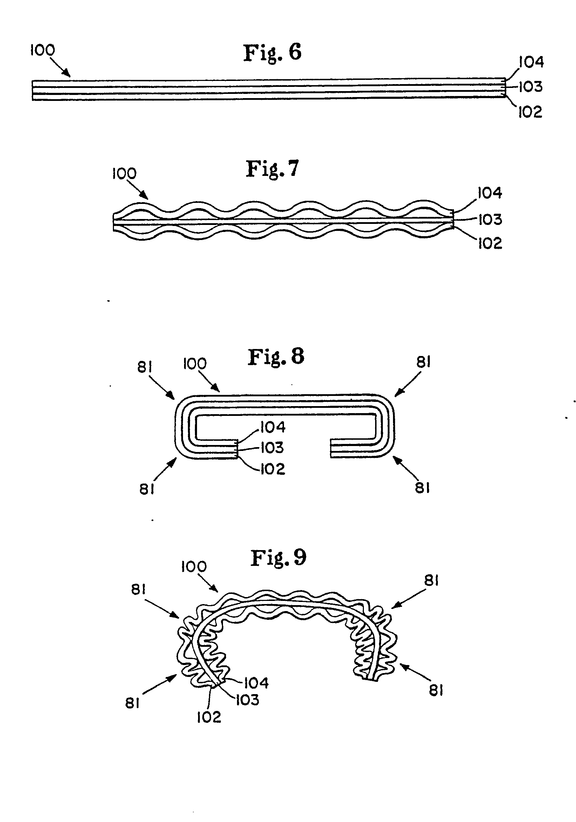

Figure 6 is an end view of a diaper 100 taken along view line 6-6 of Figure 10. In this view, the thicknesses of the three elements have been exaggerated for clarity.

Figure 7 is an end view of a diaper 10O, as shown in Figure 6, but represents diaper 100 after elastomeric material strip 103 has been subjected to sufficient heating to cause it to contract predominantly uniaxially along its length. It can be readily seen that the contraction of elastomeric material strip 103 gathers, or shirrs, topsheet 102 and backsheet 104 more or less uniformly over their entire common lengths. This is what one obtains if one applies the heating to the diaper while the diaper is in a substantially planar condition.

If the garment to which the elastomeric material is attached is a disposable diaper comprising an absorbent core, and if the diaper is folded into a C-shaped cross-section while the elastomeric material is in its heat unstable form, the elastomeric material wiil contract nonuniformly when heated. Nonuniform contraction results in (1) nonuniform gathering of the materials attached to the elastomeric material; (2) a tendency of the diaper to retain a configuration analogous to its folded configuration; and (3) a decreased tendency to waistband rollover when the diaper is worn, thereby providing better fit and decreased leakage around the waistband.

Figure 8 is an end view of a diaper 10O, as shown in Figure 6, except that diaper 100 has been folded into a C-fold configuration and is held in that configuration by holding means not shown. Diaper 100 has four fold areas 81, as illustrated. The C-fold shown in this figure is an open C-fold. Closed folds where topsheet 102 actually contacts itself on the inner surfaces of the "C" are not only possible, but also are preferred in many situations.

Figure 9 is a view of the diaper 100 shown in Figure 8, except this view is taken after elastomeric material strip 103 has been subjected to sufficient heating to cause it to substantially contract to its heat stable and elastic state. In Figure 9, the holding means associated with, but not shown in, Figure 8, has been removed. It is to be noted that disposable diaper 10O retains a general "C" configuration, athough not nearly so sharply defined as the C-fold configuration shown in Figure 8. Further, it is to be noted that neither topsheet 102 nor backsheet 104 is uniformly gathered as shown in Figure 7, They are, in fact, more gathered, or shirred, in fold regions 81 than in regions outside fold regions 81.

While expressing an intention not to be bound by the following comments, it can be theorized that the differences between the diaper structure of Figure 7 and the diaper structure of Figure 9 flow from a tendency of the absorbent core to resist the contraction of the elastomeric material in those regions where the absorbent core is intact. The bending of the absorbent core, as at the folds, tends to decrease the amount of resistance the core engenders, thereby allowing the elastomeric material to contract more fully in the regions associated with the folds.

The elastomeric materials useful in the present invention include a number of materials well known to those skilled in the art. For example, the polyurethane described in the hereinbefore mentioned patent to Koch et al. and the plasticized vinyl chloride described in the hereinbefore mentioned patent to Massengale et al. can be used. Further, the compositions comprising a mixture of a thermoplastic resin material (or other organic, normally solid heat flowable material) in an elastomeric material as described by Cook in U.S. Patent Re 28,688, can also be used.

One particularly preferred elastomeric material useful herein is a film comprising a blend of ethylene propylene rubber with ethylene vinyl acetate, such as that available from Exxon Chemical Company of Florham Park, New Jersey, Specific film-forming thermoplastic elastomeric polymer compositions which are described as being suitable for forming webs capable of exhibiting heat stable and eat unstable states are disclosed in U.S. Patent 4,303,571 issued to Jansen et al, on December 1, 1981.

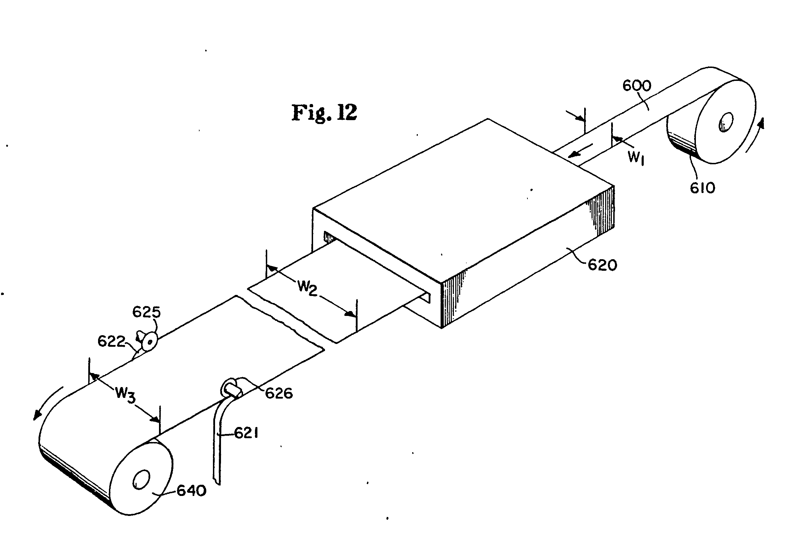

The film is preferably converted to a dimensionally heat unstable state via molecular orientation by laterally stretching it while at an elevated temperature which is less than its crystalline melting temperature and allowing it to cool. This is generally done by means of a tentering apparatus, i.e., an apparatus designed to stretch the film in a direction perpendicular to the direction of web travel, as generally shown in Figure 12.

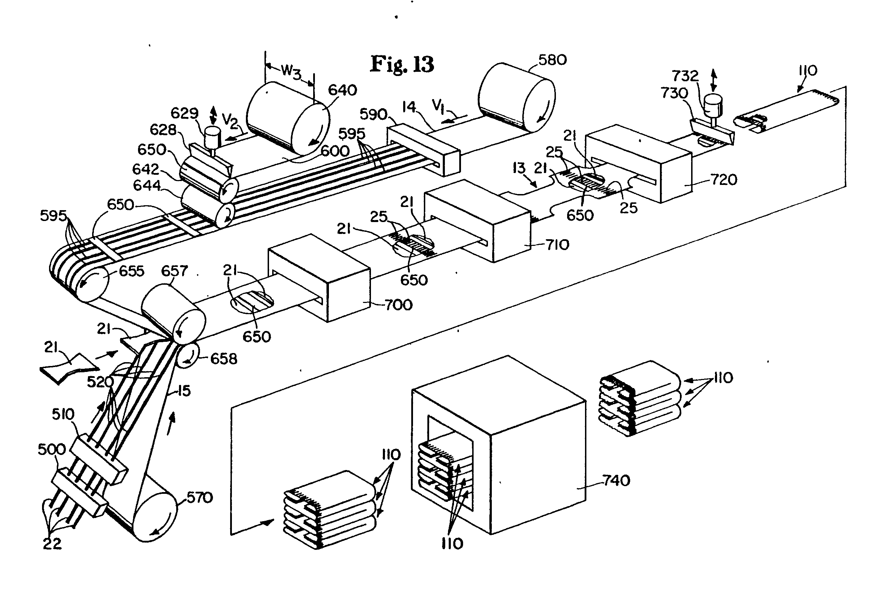

A typical tentering operation is schematically illustrated in Figure 12. A web 600 of heat stable elastomeric material is fed from a roll 610 into a heated tentering apparatus 620. The temperature of the tentering apparatus is below the crystalline melting temperature of the material. For materials such as the blend of ethylene propylene rubber and vinyl acetate mentioned earlier herein, the tentering apparatus is preferably maintained at a temperature of approximately 65°C. After softening, the web 600 is stretched from an initial width $W_1$, to approximately four times its initial width, as measured in a direction perpendicular to its direction of travel, i.e., as measured in the cross-machine direction. The stretched film is then allowed to cool to ambient temperature either with or without restraint. When cooled, the polymers are predominantly uniaxially cross-machine direction oriented and the film essentially retains its new dimension until such time as it is again subjected to an elevated temperature. The film is thus in a heat unstable state. (It should be noted that the material may still be elastic, even in its heat unstable state.) Because the outermost edges 621, 622 of the web 600 are clamped during the lateral stretching operation, they do not become stretched. Accordingly, the outermost edges 621, 622 of the heat unstable web 600 are preferably trimmed from the web by means of knives 625, 626 upon discharge form the tentering apparatus 620. This reduces the heat unstable web from a width $W_2$ to a final width $W_3$ prior to winding onto a roll 640. As will be pointed out hereinafter, the final width $W_3$ of the roll of heat unstable elastomeric material preferably corresponds to the overall length of the discrete elastically contractible segments cut from the web. It is of course recognized that a web of significantly greater width than that needed for a single elastomeric element could be prepared and thereafter slit into multiple rolls of the desired width. The elastically contractible segments are preferably applied in a substantially untensioned condition with their lengths oriented in a direction substantially perpendicular to the direction of web travel in the manner generally illustrated in Figure 13. Subsequent treatment of the discrete articles cut from the web, as with heated air, at about 68°C is normally sufficient to cause the elastically contractible elements to shrink in a direction parallel to their length and reassume a dimensionally heat stable and elastic state.

Figure 13 schematically discloses a particularly preferred method of practicing the present invention to produce a disposable diaper 110 of the type generally illustrated in Figure 11. The method is generally in accordance with the teachings of the aforementioned U.S. Patent 4,081,301 issued to Buell on March 28, 1978. In particular, a web of plastic backsheet material 14 is fed from a supply roll 580 at velocity $V_1$ to a glue applicator, shown schematically as 590, where a multiplicity of adhesive stripes 595 are applied to the uppermost surface of the web. A roll 640 of heat unstable elastomeric material 600 prepared generally in accordance with Figure 12 and having an overall width $W_3$ is fed at a second velocity $V_2$ which is less than the velocity $V_1$ of backsheet web 14. Vacuum roll 642 and transfer roll 644 operate at peripheral velocities corresponding to the velocity $V_1$ of backsheet web 14. Accordingly, there is slippage between those portions of heat unstable elastomeric web 600 in contact with the periphery of vacuum roll 642 and vacuum roll 642.

Cutting knife 628 operated by cylinder 629 is timed to cut dimensionally heat unstable elastomeric segments 650 from the moving web 600. As can be seen from Figure 13, the elastomeric segments 650 are spaced from one another by the faster moving suction roll 642 and transfer roll 644. The discrete segments 650 are held in contact along the periphery of suction roll 642 by means of vacuum applied to the interior surface of the roll and are transferred to transfer roll 644 by means of a positive pressure air blast at the point of tangency between the rolls. The transferred segment 650 is secured about the periphery of transfer roll 644 by means of vacuum until it approaches the point of tangency with moving backsheet web 14.

At the point of tangency, a positive pressure air blast is used to transfer the heat unstable elastomeric segments 650 across the small gap between the transfer roll 644 and the moving backsheet web 14. The beads of adhesive 595 utilized to assemble the various components of the diaper serve to hold the elastomeric segments 650 such that their overall length is oriented substantially perpendicular to the direction of web travel until such time as the segments are bonded at spaced locations along their length to the topsheet 15 and backsheet 14 by means of bonding apparatus 700. Bonding apparatus 700 preferably employs ultrasonic bonding procedures to provide regions of securement 25 between the segments 650 at spaced locations along their length and the topsheet 15 and backsheet 14.

For given diameters of suction roll 642 and transfer roll 644, the width of segments 650, as measured in a direction parallel to their direction of travel, and their spacing along the length of backsheet web 14 is controlled by adjusting the velocity $V_2$ of heat unstable elastomeric web 600 in relation to the velocity $V_1$ of backsheet web 14. In a typical embodiment, the speed $V_2$ of elastomeric web 600 is approximately 1/8 the speed $V_1$ of backsheet web 14.

As can be seen in Figure 13, a web of topsheet material 15 is fed from a supply roll 570 to the nip formed between a pair of combining rolls 657 and 658 along with the backsheet web 14 having spaced elastomeric segments 650 attached thereto. Shaped absorbent pad elements 21 are fed into the nip in timed relation to the spaced elastomeric segment 650. The four elastic bands 22 utilized to provide elasticized leg cuffs are fed through suitable tensioning means 500, as generally disclosed in the aforementioned U.S. Patent 4,081,301 to Buell, and adhesive 520 is applied to the stretched bands in spaced relationship to the absorbent pads 21 and elastomeric segment 650 by means of glue applicator 510. Upon passage through the nip between rolls 657 and 658, a laminate diaper web is formed.

As can be seen from the partially broken away web segment following rolls 657 and 658, each heat unstable elastomeric segment 650 is positioned intermediate adjacent absorbent pad elements 21. The laminate web is thereafter passed through a suitable securement apparatus 700, which preferably comprises ultrasonic bonding means, wherein the heat unstable elastomeric segments 650 are bonded to the backsheet 14 and topsheet 15 of the diaper web to form regions of securement 25. This is best illustrated in the partially broken out segment of the web located downstream of securement apparatus 700.

Following securement of the elastomeric segments 650 to the backsheet 14 and topsheet 15, the diaper web is thereafter passed into a side notching apparatus 710 wherein side notches are cut from the assembled web adjacent the narrow portion of each absorbent pad elements 21 to provide a crotch portion 13 in each of the interconnected disposable diapers. The web is thereafter preferably passed to a C-folding apparatus 720 wherein its cross-section is made to approximate that generally shown in Figure 8. The C-folded web is thereafter preferably cut into discrete diapers 110 by means of knife 730 actuated by cylinder 732. The cutting operation is timed in relation to movement of the web such that each elastomeric segment 650 is cut essentially in half. One half of the severed, heat unstable, elastomeric segment 650 forms the rear waistband of a first diaper while the other half of the heat unstable elastomeric segment forms the front waistband of the adjacent diaper.

The discrete diapers 110 are thereafter preferably folded about their midpoints, as shown in Figure 13, stacked upon one another for cartoning and thereafter passed into a heating tunnel 740 in a substantially untensioned state. The heat applied in the tunnel elevates the temperature of the dimensionally heat unstable elastomeric segments contained in the diapers to a point below the crystalline melting temperature of the elastomeric segments and causes the segments to return to a dimensionally heat stable and elastic condition, thereby producing a shirred or gathered waistband of the type generally illustrated in Figure 11.

Although it is possible in the practice of the present invention to perform the final heating step on the continuous web prior to cutting the interconnected diapers from one another, it has generally been found that maximum shrinkage of the elastomeric segments occurs while they are in an untensioned, i.e., unrestrained condition, particularly in the direction of shrinkage. Cutting the diapers from the web removes any possible tension applied in the direction of web travel. Since tensile forces applied perpendicular to the direction of shrinkage can, at least to a degree, inhibit shrinkage of the elastomeric segments it is generally preferred that the web be under little or no tension during the final heating step if the latter approach is employed.

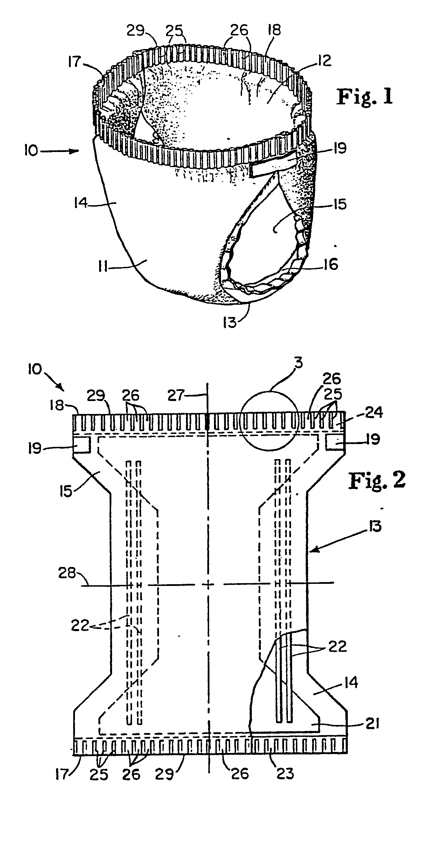

An alternative diaper embodiment produced utilizing the method schematically illustrated in Figures 12 and 13 is shown in Figures 1 through 5.

The diaper embodiment illustrated in Figure 1 is based on the disposable diaper design generally taught in the hereinbefore mentioned U.S. Patent 3,860,003 issued to Buell. The disposable diaper 10 is shown in perspective in a configuration as if it were applied about an infant. Disposable diaper 10 comprises a front portion 11 and a rear portion 12 with a crotch portion 13 interposed therebetween. In use, crotch portion 13 is placed between the legs of the infant and front portion 11 and rear portion 12 are placed, respectively, along the front and rear lower portions of the wearer's trunk. Topsheet 15 forms the inner surface of disposable diaper 10 while backsheet 14 forms its outer surface. Side flaps (or leg cuffs) 16 fit about the wearer's thighs. In use, front waistband 17 and rear waistband 18 are placed adjacent the wearer's waist regions on, respectively, the front and rear portions of the wearer's trunk. Disposable diaper 10 is held in position about the wearer by fastening tape 19. Outer margin of waistband 29 is shown in Figure 1 as the upper edge of disposable diaper 10. Transverse regions of securement 25 and transverse regions of nonsecurement 26 in the waistbands are discussed more fully hereinafter.

Figure 2 is a partially cut away plan view of disposable diaper 10 opened out into a planar configuration. Topsheet 15 is, in this illustration, the upper surface of the diaper while backsheet 14 is the lower surface. Absorbent element 21 is interposed between topsheet 15 and backsheet 14.

As illustrated, disposable diaper 10 is generally symmetrical about longitudinal centerline 27 and lateral centerline 28. While this is a preferred configuration, it is not necessary that disposable diaper 10 be symmetrical. An asymmetric orientation about lateral centerline 28, as when crotch portion 13 is transposed toward front waistband 17, is quite useful.

Disposable diaper 10 is provided with elastic members 22 in the side margins thereof running generally parallel to longitudinal center line 27. In the embodiment illustrated, two elastic members 22 are placed on either side of disposable diaper 10; single or multiple elastic members can be used. These elastic members may, if desired, be applied in the direction of web travel while in a stretched condition in accordance with the teachings of U.S. Patent 4,081,301 issued to Buell on March 28, 1978.

Fastening tapes 19 are secured to disposable diaper 10 adjacent rear waistband 18.

Front waist elastomeric element 23 and rear waist elastomeric element 24 are positioned, respectively, in front waistband 17 and rear waistband 18 adjacent outer margin of waistband 29. In the embodiment illustrated in Figures 1 and 2, disposable diaper 10 comprises elastic waist elements in both the front and the rear waistbands.

Transverse regions of securement 25 and transverse regions of nonsecurement 26 are also illustrated in Figure 2.

One major function of backsheet 14 is to prevent body fluids from escaping from disposable diaper 10 and soiling the wearer's outer garments and other surfaces in contact with the disposable diaper. Any compliant, non-irritating, substantially planar material which is impermeable to body fluids can be used as backsheet 14. Suitable materials are described with particularity in the hereinbefore mentioned patents. A preferred backsheet is formed from polyethylene film having a thickness of from 0.012 millimeter (mm) to 0.051 millimeter (mm).

Breathable backsheets (i.e., backsheets that permit the passage of vapor and air while retarding the passage of liquid) useful in the present invention are known in the art. They are typically comprised of apertured film.

The size of backsheet 14 is dictated by the exact diaper design selected and the size of the infant intended to be the wearer.

Topsheet 15 can be any compliant, soft feeling, non-irritating (to the wearer's skin), substantially planar material. It functions to contact the wearer's skin, to receive fluid discharges, to allow the discharges to pass readily therethrough into the absorbent element, and to isolate the wearer's skin from the fluids in the absorbent element. To aid in effective performance of the last function, the topsheet is preferably hydrophobic.

Topsheet 15 can be porous paper made from natural or synthetic fibers or mixtures thereof, non-woven fabric made from natural or synthetic fibers or mixtures thereof, apertured plastic film, porous foam, or the like. Examples of suitable topsheets are described in the hereinbefore incorporated patents.

A preferred topsheet is spun bonded non-woven polyester fabric made from fibers of from 0.242 to 0.275 Tex having a basis weight of 17 grams (g) per square meter ($M^2$). Another preferred topsheet material has a basis weight of 22 g per $M^2$ and comprises 65% (by weight) staple length, 0.16 Tex polyester fibers (such as Kodel type 411 polyester fibers as sold by Tennessee Eastman Corporation, Kingsport, Tennessee); 15% crimped, staple length, 0.16 Tex rayon fibers; and 20% acrylic copolymer binder (such as Celanese CPE 8335 as sold by Celanese Corporation of Charlotte, North Carolina)."Staple length", as used herein, refers to fibers having a length of at least 15 mm.

Still another preferred topsheet is constructed from polypropylene fibers which have been carded and thermally bonded in a spaced-apart pattern, Fibers 3.8 centimeters (cm) long and of from 0.165 to 0.33 Tex are suitable. A preferred topsheet of this type has a basis weight of 24 g per $M^2$.

Suitable topsheets can also be constructed from macroscopically expanded, three-dimensional apertured plastic films such as those described by Radel and Thompson in U.S. Patent 4,342,314, issued August 3, 1982; Ferguson and Landrigan in U.S. Patent 4,341,217, issued July 27, 1982; and Thompson in U.S. Patent 3,929,135, issued December 30, 1975.

As with the case of backsheet 14, the size of topsheet 15 is dictated by the exact diaper design selected.

Absorbent element 21 can be comprised of any material which is generally compressible, conformable and which is capable of absorbing and retaining fluids.

Absorbent element 21 can be constructed from any of a variety of materials commonly used in disposable absorbent articles and which are described in the hereinbefore incorporated patents. Examples of suitable absorbent materials include creped cellulose wadding, absorbent foams, absorbent sponges, super absorbent polymers, and, preferably, comminuted and airlaid wood pulp fibers commonly referred to as either absorbent fluff or airfelt. An absorbent fluff having a density of from 0,05 to 0.175 g per cm³ is generally acceptable.

As in the case of backsheet 14 and topsheet 15, the size of absorbent element 21 is dictated by the exact diaper design selected.

Optionally, absorbent element 21 can have associated with either or both planar faces envelope tissues (not illustrated in the drawings) comprising any permeable material well known to those skilled in the art, such as wet strength tissue paper. When used, envelope tissues are generally coextensive with absorbent element 21 and either coterminous therewith or folded up and about the laterally extending margins thereof. Envelope tissues can optionally be secured to absorbent core 21 by any means well known to those skilled in the art.

Absorbent element 21 is interposed between backsheet 14 and topsheet 15. The diaper design selected determines whether or not the three elements are coterminous although, in general, either backsheet 14 or topsheet 15 or both extend beyond the margins of absorbent element 21. In the diaper embodiment illustrated in Figures 1 through 5, both backsheet 14 and topsheet 15 preferably extend beyond the laterally extending margins of absorbent element 21 and are essentially coterminous along their laterally extending margins.

Optionally, backsheet 14 can be secured to absorbent element 21 by any convenient means (not illustrated in the drawings) well known to those skilled in the art. Examples of suitable means are parallel beads of adhesive (such as hot melt adhesive) and double sided adhesive tape; each extend essentially the entire longitudinal length of absorbent element 21.

Elastic members 22 serve to contract or gather the cuffs (longitudinally extending margins) of disposable diaper 10 and maintain them in contact with the legs of the wearer, thereby providing improved fit and reducing fluid leakage from the diaper. One material which can be used for elastic elements 22 is an elastic tape having a cross section of 0.18 mm by from 1.5 mm to 6.4 mm and made from natural rubber, as available from East Hampton Rubber Company of Stuart, Virginia, under the trademark L-190O Rubber Compound. Other suitable elastic members can be made from natural rubber elastic tapes sold under the trademarks Fulflex 9211 and Fulflex 9111 by Fulflex Company of Scotland, North Carolina.

The length of elastic elements 22 is dictated by the precise diaper design chosen. In the design illustrated in Figures 1 and 2, elastic elements 22 extend a major portion of the longitudinal length of disposable diaper 10, but terminate outside the waist regions of disposable diaper 10.

Elastic members 22 are operably associated with disposable diaper 10 by securing them to the diaper adjacent its longitudinally extending margins by elastic attachment means which are not shown in the Figures. The elastic attachment means should be flexible and of sufficient adhesiveness to hold elastic members 22 in their stretched condition substantially indefinitely. One suitable means is hot melt adhesive. A more detailed description of the manner in which the majority of components comprising the diaper, including the elastic members 22 should be positioned and secured to form a disposable diaper of the type generally shown herein is given in the hereinbefore mentioned U.S. Patent 4,081,301 issued to Buell on March 28, 1978.

Elastic members 22 are affixed to disposable diaper 10 in an elastically contractible condition so that in a normally unrestrained configuration elastic members 22 effectively contract or gather the diaper material adjacent elastic members 22. Elastic members 22 can be affixed to disposable diaper 10 in an elastically contractible condition in at least two ways. For example, elastic members 22 can be stretched to an elongated orientation and affixed to disposable diaper 10 while disposable diaper 10 is in an uncontracted condition. Alternatively, disposable diaper 10 can be contracted (in crotch portion 13, for example by pleating) and elastic members 22 can be affixed to the contracted disposable diaper 10 while the elastic members are in their relaxed or unstretched orientation.

Front waist elastomeric element 23 and rear waist elastomeric element 24 are each formed of elastomeric material, as hereinbefore described. Waist elastomeric elements 23 and 24 are preferably at least 0.6 cm wide, most preferably at least 1.6 cm wide. While the maximum width of waist elastomeric elements 23 and 24, as measured in a direction parallel to the direction of web travel during the processing operation schematically illustrated in Figure 13, is determined by the diaper design and matters of economy, they generally are no wider than 3.8 cm.

In the diaper embodiment illustrated in Figures 1 and 2 waist elastomeric elements 23 and 24 each extend across essentially the entire lateral width of disposable diaper 10. They are preferably applied in accordance with the continuous web assembly operation generally disclosed in Figure 13. While this is a preferred construction, the present invention may be employed to produce diaper structures wherein waist elements 23 and 24 extend across only a portion of the lateral width of the diaper.

The topsheet, backsheet, and each waist elastomeric element are affixed together by transverse regions of securement while the waist elastomeric elements are in their heat unstable state and all three elements are essentially fully, but not elastically, extended. The system is later heated (as with heated air) and the waist elastomeric element is allowed to return to its heat stable and elastic state.

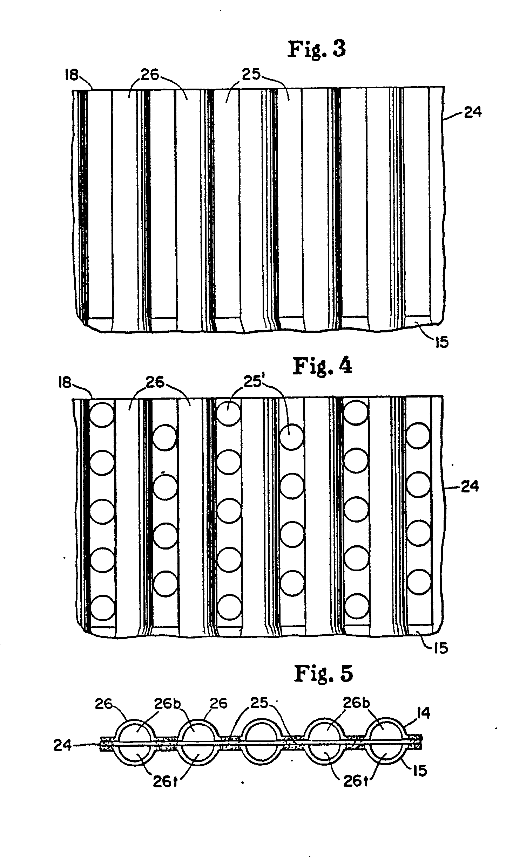

Transverse regions of securement 25 are shown in a generalized representation in Figures 1 and 2, More specific embodiments of transverse regions of securement 25 are depicted in Figures 3 and 4 which are enlarged views of a portion of rear waistband 18 indicated by reference numeral 3 in Figure 2. In these views, the elastomeric material is in its heat stable and elastic state.

In this discussion of Figures 3 and 4, reference shall be made to rear waistband 18 and the components thereof. The same comments can be made about front waistband 17 and its components.

Transverse regions of securement 25 extend essentially across the whole width of waist elastomeric element 24. The term "transverse" as used in this context refers to an orientation generally perpendicular to the major laterally extending dimension of waistband 18, i.e., it corresponds to the direction of web travel generally illustrated in both Figures 12 and 13. That is to say, since rear waistband 18 extends laterally across the width of disposable diaper 10 and is generally parallel to lateral centerline 28, the transverse regions of securement 25 extend across rear waistband 18 in an orientation essentially parallel to longitudinal centerline 27; they are directed generally from outer margin of waistband 29 to the center of disposable diaper 10. It should be noted that the transverse regions of securement 25 are prefer-ably spaced and positioned so that they do not interfere with the elastic bands 22 when the bands are in a continuous, stretched condition, as generally illustrated in Figure 13. This permits the unsecured portions of the stretched bands 22, i.e., those portions generally coinciding with the waistband, to retract to the position generally illustrated in Figure 2 upon severance of the diapers from the web. As also illustrated, transverse regions of securement 25 are shown to be at essentially right angles to lateral centerline 28 and to the lateral extent of waistband 18. This is the preferred orientation.

The term "essentially across" is used in this context to indicate that transverse regions of securement 25 need not extend absolutely across the entire width of waist elastomeric element 24. It is, however, preferable that they extend sufficiently far across the width thereof to provide the channels discussed hereinafter.

In Figure 3, transverse regions of securement 25 are shown as essentially regularly spaced unitary zones of sealing attaching waist elastomeric element 24 to topsheet 15 and backsheet 14 which is not visible in Figure 3 or 4. The precise means for providing the zones of sealing can be readily selected by those skilled in the art. Examples include adhesive attachment, solvent sealing and the like. Preferably, ultrasonic welding is used.

As illustrated in Figures 3, 4, and 5, the points of attachment of both topsheet 15 and backsheet 14 to waist elastomeric element 24 are in register (i.e, are coextensive). This is a preferred orientation, but the points of attachment of topsheet 15 to waist elastomeric element 24 can be offset from the adjacent points of attachment of backsheet 14 to waist elastomeric element 24. In such a situation there will be offset transverse regions of securement on either side of the waist elastomeric element.

Figure 4 illustrates an alternate embodiment of transverse regions of securement 25'. In this embodiment, the transverse regions of securement comprise discrete spaced zones of sealing, preferably ultrasonic welds, effectively attaching the materials together and forming the channels hereinafter described. Preferably the discrete spaced zones are circular or elliptical.

Transverse regions of securement 25 are preferably from 0.15 to 1.0 cm wide (i.e. in the dimension generally parallel to lateral centerline 28. They are also preferably regularly spaced, but they can be nonuniformly spaced. They are most preferably from 0.3 to 1.5 cm apart as measured from center to center.

Figure 5 illustrates the functioning of the transverse regions of securement. Figure 5 is an end view of the rear waistband 18, typical of the alternate embodiments shown in Figures 3 and 4, with rear waist elastomeric element 24 in its heat stable and elastic state. In Figure 5, transverse regions of securement 25 are shown as darkened portions for emphasis. Topsheet 15 and backsheet 14 are shown gathered. These gathers constitute and define transverse regions of nonsecurement 26b between backsheet 14 and rear waist elastomeric element 24 and transverse regions of nonsecurement 26t between topsheet 15 and rear waist elastomeric element 24. These transverse regions of nonsecurement 26b and 26t form open gathers or channels from the margin of the diaper extending to the interior of the diaper and terminating in the region adjacent the laterally extending edges of absorbent element 21. These open channels allow the diaper to breathe by allowing the exchange of air and vapor between the interior of the diaper and the surrounding atmosphere, even when the diaper is secured about an infant.

At the same time as transverse regions of nonsecurement 26b and 26t are formed, topsheet 15 and backsheet 14 form structures in the nature of corrugations. These corrugations which extend transversely across the width of rear waistband 18 tend to stiffen the waistband, thereby tending to prevent waistband rollover (i.e., the bending of the waistband about itself).

After severance from the web, the diaper illustrated in Figure 1 is heated to cause the waistband elastomeric elements 23 and 24 to change from their heat unstable to their heat stable state. In the embodiment shown in Figure 1, the heat is applied while the diaper is in an untensioned, generally planar configuration. As illustrated in Figure 1, relatively uniform corrugations and channels were produced.

By way of contrast, the diaper 110 is made as the diaper of Figure 1, but heat treated while in an untensioned, C-folded configuration. The result produced is shown in Figure 11. In particular, before heating the diaper to cause the waist elastomeric elements to change from their heat unstable state to their heat stable and elastic state, the front and rear waist regions 17 and 18 were folded into a closed C-fold configuration, as indicated in Figure 8, and severed from the web, as generally taught in the aforementioned U.S. Patent 4,081,301 to Buell.

Following the heating operation in the C-folded configuration, which converted the waist elastomeric elements to their heat stable and elastic state, front waistband 17, rear waistband 18, and diaper 110 exhibited a generally shaped configuration, even in the absence of securement with adhesive tapes 19. The size of the corrugations and channels was not uniform with the corrugations and channels being smaller in regions of fold 81.

As indicated hereinbefore, the diaper embodiments described in detail in conjunction with the present method invention are intended to be illustrative of articles to which the present invention has particular application. Other elasticized articles produced in continuous web form and embodying elastically contractible elements which tend to cause shirring in a direction substantially perpendicular to the direction of web travel can be produced with equal facility by applying the teachings herein contained.

In the foregoing discussion, the elastomeric waistband material which was affixed to the diaper in a heat unstable state and was then treated to cause it to be transformed into a heat stable and elastic state has been discussed in terms of a "heat unstable" elastomer. More broadly, the elastomeric materials useful herein are those materials that have a dimensionally unstable state relative to some other dimensionally stable and elastic state and which can be caused to be transformed from the unstable to the stable state by the application of any form of energy or by any other convenient treatment. The most convenient and the most practical form of energy is heat, and the materials are described in such terms herein.

Still further, the present invention has been described in terms of an elastomeric material which is affixed to the diaper in its unstable state and is later transformed to its stable and elastic state. While, in general, the stable state is an absolute state, it is not necessary that it be. It is only required that the state following treatment be relatively more stable than the state preceding treatment and that the state following treatment be sufficiently stable for practical use. It is, of course, necessary that the material be elastic in its stable state.

### Example

Exemplary diapers produced according to the method of the present invention are constructed following the basic design described in the aforementioned U.S. Patent 3,860,003 issued to Buell on January 14, 1975.

The diaper design hereinafter described provides for both front and rear elasticized waistbands.

The absorbent element comprises absorbent fluff having a density of 0.09 g per cm³ and a basis weight of 1100 g per $M^2$ in the crotch portion and 350 g per $M^2$ near the waist portions. It is generally hourglass-shaped and is 38.7 cm long, 25.4 cm wide at each of its laterally extending margins and 9.6 cm wide in the crotch portion. It is symmetrical about its longitudinal centerline, but asymmetrical about its lateral centerline in that the crotch portion is centered 21.6 cm from the rear lateral margin.

The topsheet comprises the thermally bonded polypropylene material hereinbefore mentioned and the backsheet 0.04 mm thick polyethylene film. Both also are hourglass-shaped and are 43.8 cm long and 30.5 cm wide at their laterally extending margins.

During construction, the absorbent element is interposed between the topsheet and the backsheet which are essentially coextensive and coterminous. Hot-melt adhesive glue beads running parallel to the longitudinal centerline secure the backsheet to the absorbent element. They also secure the backsheet to the topsheet in the cuff regions.

The cuff portions of the diapers are elasticized by incorporating therein two elastic members in each longitudinally extending margin of the diaper at the crotch portion. Each is made of Fulflex 9211 and is 2.4 mm wide and 0.18 mm thick; their relaxed length is 19.6 cm. They are extended to 220% of their original lengths at the time of attachment generally in accordance with the teachings of the aforementioned U.S. Patent 4,081,301 to Buell. These elastic members are centered about the crotch portion. The pattern defined by the pair of members is centered 9.4 cm from the longitudinal centerline of the diaper and parallel thereto. The two elastic members in each diaper margin are centered on parallel lines 1.6 cm apart.

Each diaper waistband extends the lateral width of the diaper and is 30.5 centimeters long; after cutting from the continuous web, each waistband is 2.5 centimeters wide in the transverse direction, i.e., as measured in a direction parallel to the direction of web travel. The waist elastomeric element used in each waistband is the hereinbefore described ethylene propylene rubber blended with ethylene vinyl acetate. Each of the elastomeric elements comprises a discrete segment of material cut from a moving web of heat unstable material which has been tentered in a direction substantially perpendicular to the direction of web travel in the manner generally shown in Figure 12. The elements are applied to the moving web of interconnected diapers while in an untensioned condition in the manner generally disclosed in Figure 13 so that the dimension corresponding to the final width $W_3$ of the web of elastomeric material 600 is aligned substantially perpendicular to the direction of web travel.

Prior to tentering in accordance with the process generally disclosed in Figure 12, the elastomeric web 600 has an initial width $W_1$ of approxi-

mately 8 inches, i.e., approximately 20.3 centimeters, and an initial thickness of approximately 6 mils, i.e., 0.15 millimeters. Lateral stretching of the web was performed using a tentering apparatus 620 of the type available from Marshall & Williams Company of Providence, Rhode Island. The tentering apparatus in question had three temperature zones, each being approximately 305 cm in overall length. The web 600 was fed in a heat stable condition from a roll 610 into the first 305 cm long zone at ambient temperature and without any lateral stretching. Approximately 1 inch, i.e., 2.54 centimeters, of outermost edges 621,622 of the web was gripped by the tentering apparatus. The central portion of the web 600 measuring approximately 6 inches, i.e., approximately 15 centimeters, in width, was subjected to lateral stretching over the last two 305 cm long sections of the apparatus 620. The last two 305 cm long sections were maintained at a temperature between 140° and 155°F, i.e., between 60°C and 68°C, and the central portion of the web was stretched from an initial width of 6 inches, i.e., 15 centimeters, to a width of approximately 24 inches, i.e., approximately 60 centimeters, as it passed through the last two 305 cm long zones of the apparatus. After discharge from the third 305 cm long zone of the tentering apparatus, the web was allowed to cool without restraint, resulting in a degree of contraction to a final width $W_2$ of approximately 382 mm including the 25 mm gripping portions of outermost edges 621,622. After cooling, the elastomeric web 600 is in a heat unstable condition. The overall thickness of the web 600 in the centrally located section averaged approximately 1.5 mils, i.e., 0.0038 millimeters. Prior to use in the process generally disclosed in Figure 13, the outermost edges 621,622, including the portions gripped by the tentering apparatus, are preferably removed by slitting, e.g., as by knives 625,626 to produce a final width $W_3$ which corresponds to the overall length of the waistband segments to be affixed to the diapers. In the exemplary embodiment herein described, the resultant width $W_3$ of the elastomeric heat unstable web 600 is approximately 30.5 centimeters.

In the foregoing example, the speed of web travel through the tentering apparatus 620 is between 610 cm and 793 cm per minute.

When attached in the manner generally shown in Figure 13, each discrete segment of heat unstable elastomeric material cut from roll 640 is 30.5 centimeters long (as measured perpendicular to the direction of web travel) and 5.0 centimeters wide (as measured parallel to the direction of web travel). Each discrete segment of heat unstable elastomeric material is secured to both the topsheet and the backsheet at spaced locations along its length (as measured perpendicular to the direction of web travel) in the manner schematically shown in Figure 13. At the time of securement, the top sheet and the backsheet are in fully extended configurations. No sealing of the heat unstable elastomeric waistband elements in the areas coinciding with the stretched elastic legbands is carried out to ensure that the legbands remain free to retract to their unstretched condition in their unadhered areas when the individual diapers are severed from the continuous web.

As will also be clear from an inspection of Figure 13, each heat unstable elastomeric segment bridges the waistband portion of adjacent interconnected diapers in the moving web. Accordingly, when the diapers are severed from the web as generally shown in Figure 13, the approximately 5 centimeter wide elastomeric segment is cut at approximately its midpoint. Half of the strip forms the rear waistband of one diaper while the other half of the strip forms the front waistband of the adjacent diaper.

In the exemplary diaper embodiment herein described, transverse regions of securement comprising discrete zones of ultrasonic welds are used to affix each waist elastomeric element to both the topsheet and to the backsheet. The transverse regions of securement each comprise seven discrete elliptical zones of ultrasonic welds each having a major axis of 1.9 mm and a minor axis of 1.0 mm; each individual ellipse is set with its major axis at an angle of 45° to the transverse direction. The transverse regions of securement are generally regularly spaced along each waistband and their centers are 6.4 mm apart.

Adhesive fastening tapes were adhesively affixed to the diaper.

Each assembled diaper is folded in a closed C-fold as described above, cut from the web, folded approximately about its centerline 28, collected into multi-unit stacks for cartoning and heated with air at 68°C so that the elastomeric material comprising waistbands 23 and 24 shrinks to its heat stable and elastic state.

## Claims

1. A continuous method for elasticizing substantially inelastic articles by securing elastically contractible elements to a moving, interconnected web of said articles, said elastically contractible elements causing the discrete articles cut from said web to shirr in a direction substantially perpendicular to the direction of web travel, said method comprising the steps of:

(a) subjecting a web of elastomeric material capable of exhibiting dimensionally heat stable and dimensionally heat unstable states to lateral stretching while said web is at an elevated temperature which is less than its crystalline melting temperature;

(b) allowing said elastomeric web to cool to ambient temperature while in a laterally stretched condition to establish a heat unstable state in the material comprising said web.

(c) feeding said web of elastomeric heat unstable material in a direction substantially parallel to said moving web of interconnected articles;

(d) severing discrete segments of said elastomeric heat unstable material from said elastomeric web in a direction substantially perpendicular to the direction of web travel, whereby each of said discrete segments of elastomeric material has its length oriented parallel to the direction of said lateral stretching and its width extending in a direction substantially parallel to the direction of web travel;

(e) transferring said discrete, severed segments of elastomeric material in a substantially untensioned condition and while their lengths are oriented substantially perpendicular to the direction of web travel to said moving web of interconnected articles at isolated, predetermined locations along the length of said web;

(f) securing said discrete segments of substantially untensioned, heat unstable elastomeric material to said web of interconnected articles at predetermined spaced locations along the length of said segments, as measured in a direction substantially perpendicular to the direction of web travel;

(g) severing each of the interconnected articles from one another, thereby forming a multiplicity of discrete articles, each having at least one segment of substantially untensioned elastomeric material in a heat unstable condition secured thereto; and

(h) heating said discrete articles to an elevated temperature which is less than the crystalline melting temperature of said heat unstable segments of substantially untensioned elastomeric material, thereby causing said elastomeric material to contract in a direction substantially parallel to its overall length, to become heat stable and elastic, and to shirr said articles in a direction substantially perpendicular to said direction of web travel.

2. A method according to claim 1, wherein the width of said web of heat unstable elastomeric material corresponds to the length of said discrete segments of elastomeric material.

3. A method according to either one of claims 1 and 2, wherein said web of interconnected articles is subjected to a folding operation aligned substantially parallel to the direction of web travel prior to severing said interconnected articles from one another, said discrete articles thereafter being subjected to said heating while they are in said folded configuration to provide non-uniform shirring in the resultant elasticized articles.

4. A method according to claim 3, wherein said web of interconnected articles is subjected to a folding operation to produce a C-shaped cross-section prior to heating.

5. A method according to any one of claims 1-4, wherein the step of transferring discrete severed segments of elastomeric material comprises feeding said web of heat unstable, substantially untensioned elastomeric material in a direction parallel to the direction of travel of said web of interconnected articles at a velocity less than that of said web of interconnected articles, and thereafter severing discrete segments from said web of elastomeric material.

6. A method according to any one of claims 1-5, wherein said discrete segments of substantially untensioned, heat unstable elastomeric material are secured to said web of interconnected articles by ultrasonic bonding.

7. A method according to any one of claims 1-6, wherein said elastomeric material comprises a blend of ethylene propylene rubber and ethylene vinyl acetate.

8. A method according to any one of claims 1-7, wherein said discrete, severed segments of heat unstable elastomeric material are indirectly transferred to said moving web of interconnected articles.

9. A method according to claim 8, wherein said indirect transfer is carried out by securing said discrete, severed segments of heat unstable elastomeric material at isolated, predetermined locations along a continuous web comprising an element of said web of interconnected articles.

10. A method according to any one of claims 1-9, wherein said elastomeric web is allowed to cool to ambient temperature after lateral stretching without restraint.

11. A method according to any one of claims 1-10, wherein the step of severing the interconnected articles from said web takes place after the heating step that causes said elastomeric material to contract in a direction substantially parallel to its overall length.

12. A method according to any one of claims 1-11, wherein the substantially inelastic articles comprise disposable diapers comprising a backsheet, an absorbent element and a topsheet, wherein an elastically contractible element is secured to at least one waistband portion of each said diaper.

**Patentansprüche**

1. Kontinuierliches Verfahren zum Elastischmachen von im wesentlichen nicht-elastischen Artikeln durch Daranbefestigen von elastisch zusammenziehbaren Elementen an einer in Bewegung befindlichen Bahn aus den genannten, miteinander verbundenen Artikeln, wobei die genannten elastisch zusammenziehbaren Elemente bewirken, daß die von der genannten Bahn abgeschnittenen, getrennten Artikel in einer Richtung gerafft werden, die im wesentlichen senkrecht zur Bewegungsrichtung der Bahn verläuft, wobei das genannte Verfahren die folgenden Stufen umfaßt:

(a) das seitliche Strecken einer Bahn aus elastomerem Material, welches befähigt ist, wärmedimensionsstabile und wärmedimensionsinstabile Zustände aufzuweisen, während sich die genannte Bahn auf einer erhöhten Temperatur befindet, die unterhalb der kristallinen Schmelztemperatur des elasto meren Materials liegt;

(b) das Abkühlenlassen der genannten elastomeren Bahn auf Umgebungstemperatur, während sie sich in einem seitlich gestreckten Zustand befindet, um in dem die genannte Bahn aufbauenden Material einen wärmeinstabilen Zustand hervorzurufen;

(c) das Zuführen der genannten Bahn aus wärmeinstabilem, elastomerem Material in einer Richtung, die im wesentlichen parallel zur genannten, in Bewegung befindlichen Bahn aus miteinander verbundenen Artikeln verläuft;

(d) das Abschneiden getrennter Abschnitte aus dem genannten wärmeinstabilen, elastomeren Material von der genannten elastomeren Bahn in einer Richtung, die im wesentlichen senkrecht zur Bewegungsrichtung der Bahn verläuft, wodurch die Längenerstreckung von jedem der genannten getrennten Abschnitte aus elastomerem Material parallel zur Richtung des genannten seitlichen Streckens ausgerichtet wird, und wodurch sich die Breite von jedem der genannten getrennten Abschnitte aus elastomerem Material in einer Richtung erstreckt, die im wesentlichen parallel zur Bewegungsrichtung der Bahn verläuft;

(e) das Übertragen der genannten abgeschnittenen, getrennten Abschnitte aus elastomerem Material in einem im wesentlichen spannungsfreien Zustand, und während die Längenerstreckungen der genannten getrennten Abschnitte aus elastomerem Material im wesentlichen senkrecht zur Bewegungsrichtung der Bahn ausgerichtet sind, auf die genannte, in Bewegung befindliche Bahn aus miteinander verbundenen Artikeln an isolierten, im voraus bestimmten Stellen längs der Längenerstreckung der genannten Bahn;

(f) das Befestigen der genannten getrennten Abschnitte aus im wesentlichen spannungsfreiem, wärmeinstabilem, elastomerem Material an der genannten Bahn aus miteinander verbundenen Artikeln an im voraus bestimmten, im Abstand voneinander angeordneten Stellen längs der Längenerstreckung der genannten Abschnitte, gemessen in einer Richtung, die im wesentlichen senkrecht zur Bewegungsrichtung der Bahn verläuft;

(g) das Auseinanderschneiden der jeweiligen, miteinander vebundenen Artikel unter Bildung einer Vielzahl von getrennten Artikeln, von denen jeder wenigstens einen Abschnitt aus im wesentlichen spannungsfreiem, elastomerem Material in einem wärmeinstabilen Zustand daran befestigt aufweist; und

(h) das Erhitzen der genannten getrennten Artikel auf eine erhöhte Temperatur, welche unterhalb der kristallinen Schmelztemperatur der genannten, wärmeinstabilen Abschnitte aus im wesentlichen spannungsfreiem, elastomerem Material liegt, wodurch bewirkt wird, daß sich das genannte elastomere Material in einer Richtung zusammenzieht, die im wesentlichen parallel zu seiner Gesamtlänge verläuft, wobei dieses elastomere Material wärmestabil und elastisch wid, und wodurch weiterhin bewirkt wird, daß die genannten Artikel in einer Richtung gerafft werden, die im wesentlichen senkrecht zu der genannten Bewegungsrichtung der Bahn verläuft.

2. Verfahren nach Anspruch 1, wobei die Breite der genannten Bahn aus wärmeinstabilem, elastomerem Material der Länge der genannten getrennten Abschnitte aus elastomerem Material entspricht.

3. Verfahren nach einem der Ansprüche 1 und 2, wobei die genannte Bahn aus miteinander verbundenen Artikeln vor dem Auseinanderschneiden der genannten, miteinander verbundenen Artikel einem Faltungsarbeitsvorgang unterworfen wird, welcher im wesentlichen parallel zur Bewegungsrichtung der Bahn erfolgt, wobei die genannten getrennten Artikel anschließend, noch während sie sich in der genannten gefalteten Konfiguration befinden, dem genannten Erhitzen unterworfen werden, um in den entstandenen, elastisch gemachten Artikeln eine nicht gleichförmige Raffung hervorzurufen.

4. Verfahren nach Anspruch 3, wobei die genannte Bahn aus miteinander verbundenen Artikeln vor dem Erhitzen einem Faltungsarbeitsvorgang unter Bildung eines C-förmigen Querschnittes unterworfen wird.

5. Verfahren nach einem der Ansprüche 1 bis 4, wobei die Stufe des Übertragens der getrennten, abgeschnittenen Abschnitte aus elastomerem Material darin besteht, daß die genannte Bahn aus wärmeinstabilem, im wesentlichen spannungsfreiem, elastomerem Material in einer Richtung zugeführt wird, die parallel zur Bewegungsrichtung der genannten Bahn aus miteinander verbundenen Artikeln verläuft, und zwar mit einer geringeren Geschwindigkeit als jener der genannten Bahn aus miteinander verbundenen Artikeln, und daß anschließend getrennte Abschnitte von der genannten Bahn aus elastomerem Material abgeschnitten werden.

6. Verfahren nach einem der Ansprüche 1 bis 5, wobei die genannten getrennten Abschnitte aus im wesentlichen spannungsfreiem, wärmeinstabilem, elastomerem Material an der genannten Bahn aus miteinander verbundenen Artikeln durch Ultraschallschweißung befestigt werden.

7. Verfahren nach einem der Ansprüche 1 bis 6, wobei das genannte elastomere Material ein Gemisch aus Ethylenpropylengummi und Ethylenvinylacetat enthält.

8. Verfahren nach einem der Ansprüche 1 bis 7, wobei die genannten abgeschnittenen, getrennten Abschnitte aus wärmeinstabilem, elastomerem Material indirekt auf die genannte, in Bewegung befindliche Bahn aus miteinander verbundenen Artikeln übertragen werden.

9. Verfahren nach Anspruch 8, wobei die genannte, indirekte Übertragung dadurch erfolgt, daß die genannten abgeschnittenen, getrennten Abschnitte aus wärmeinstabilem, elastomerem Material an isolierten, im voraus bestimmten Stellen längs einer kontinuierlichen Bahn befestigt werden, welche Stellen jeweils ein Element der genannten Bahn aus miteinander verbundenen Artikeln umfassen.

10. Verfahren nach einem der Ansprüche 1 bis 9, wobei die genannte elastomere Bahn nach dem seitlichen Strecken ohne Beschränkung auf Umgebungstemperatur abkühlen gelassen wird.

11. Verfahren nach einem der Ansprüche 1 bis 10, wobei die Stufe des Abschneidens der miteinander verbundenen Artikel von der genannten Bahn nach der Erhitzungsstufe erfolgt, welche letztere bewirkt, daß sich das genannte elastomere Material in einer Richtung zusammenzieht, die im wesentlichen parallel zu seiner Gesamtlänge verläuft.

12. Verfahren nach einem der Ansprüche 1 bis 11, wobei die im wesentlichen nicht-elastischen Artikel Wegwerfwindeln mit einer Unterlage, einem absorbierenden Element und einer Abdecklage umfassen, in welchen ein elastisch zusammenziehbares Element an wenigstens einem Taillenbandteil einer jeden derartigen Windel befestigt ist.

## Revendications

1. Un procédé continu pour rendre élastiques des articles essentiellement non élastiques en fixant des éléments élastiquement contractables sur une bande mobile desdits articles interconnectés, lesdits éléments élastiquement contractables amenant les articles discrets coupés de ladite bande à froncer dans une direction à peu près perpendiculaire à la direction du mouvement de la bande, ledit procédé comprenant les étapes suivantes:

a) soumettre une bande de matière élastomère susceptible de présenter des états stables à la chaleur et des états instables à la chaleur au point de vue dimensionnel pour l'étirer latéralement pendant que ladite bande est à une température élevée qui est inférieure à sa température de fusion cristalline,

b) permettre à ladite bande élastomère de se refroidir à la température ambiante alors qu'elle est en condition latéralement étirée pour obtenir un état instable à la chaleur dans la matière constituant ladite bande,

c) faire avancer ladite bande de matière élastomère instable à la chaleur dans une direction à peu près parallèle à ladite bande mobile d'articles interconnectés;

d) détacher des segments discrets de ladite matière élastomère instable à la chaleur de ladite bande élastomère dans une direction à peu près perpendiculaire à la direction du mouvement de la bande, grâce à quoi chacun desdits segments discrets de matière élastomère a sa longueur orientée parallèlement à la direction dudit étirage latéral et sa largeur s'étendant dans une direction à peu près parallèle à la direction de mouvement de la bande;

e) transférer lesdits segments discrets, découpés de matière élastomère dans une condition à peu près détendue et pendant que leurs longueurs sont orientées à peu près perpendiculairement à la direction du mouvement de la bande vers ladite bande mobile d'articles interconnectés en des endroits prédéterminés séparés le long de la longueur de ladite bande;

f) fixer lesdits segments discrets de matière élastomère instable à la chaleur à peu près non étirée à ladite bande d'articles interconnectés en des endroits prédéterminés, espacés le long desdits segments, tels que mesurés dans une direction à peu près perpendiculaire à la direction du mouvement de la bande;

g) découper chacun des articles interconnectés les uns des autres de manière à former une multiplicité d'articles discrets, ayant chacun au moins un segment de matière élastomère à peu près non étirée dans une condition instable à la chaleur fixé à celle-ci, et,

h) chauffer lesdits articles discrets à une température élevée qui est inférieure à la température de fusion cristalline desdits segments instables à la chaleur de la matière élastomère à peu près non étirée, de manière à amener ladite matière élastomère à se contracter dans une direction à peu près parallèle à sa longueur totale, pour l'amener à l'état stable et élastique et à froncer lesdits articles dans une direction à peu près perpendiculaire à ladite direction du mouvement de la bande.

2. Un procédé selon la revendication 1, dans lequel la largeur de ladite bande de matière élastomère instable à la chaleur correspond à la longueur desdits segments discrets de matière élastomère.

3. Un procédé selon l'une quelconque des revendications 1 et 2, dans lequel ladite bande d'articles interconnectés est soumise à une opération de formation de plis alignés à peu près parallèlement à la direction du mouvement de la bande avant de découper lesdits articles interconnectés les uns des autres, lesdits articles discrets étant ensuite soumis audit chauffage alors qu'ils sont dans leur forme plissée pour assurer un froncement non uniforme dans les articles résultants rendus élastiques.

4. Un procédé selon la revendication 3 dans lequel ladite bande d'articles interconnectés est soumise à une opération de formation de plis pour produire une section transversale en forme de C avant le chauffage.

5. Un procédé selon l'une quelconque des revendications 1-4, dans lequel l'étape de transfert des segments découpés discrets de matière élastomère consiste à faire avancer ladite bande de matière élastomère instable à la chaleur à peu près non étirée dans une direction parallèle à la direction du mouvement de ladite bande d'articles interconnectés à une vitesse inférieure à celle de ladite bande d'articles interconnectés et à découper ensuite des segments discrets de ladite bande de matière élastomère.

6. Un procédé selon l'une quelconque des revendications 1-5, dans lequel lesdits segments discrets de matière élastomère instables à la chaleur à peu près non étirés sont fixés à ladite bande d'articles interconnectés par soudage aux ultrasons.

7. Un procédé selon l'une quelconque des revendications 1-6, dans lequel ladite matière élastomère est constituée d'un mélange de caoutchouc d'éthylène-propylène et d'acétate de vinyle et d'éthylène.

8. Un procédé selon l'une quelconque des revendications 1-7, dans lequel lesdits segments discrets découpés de matière élastomère instable à la chaleur sont transférés indirectement vers ladite bande mobile d'articles interconnectés.

9. Un procédé selon la revendication 8, dans lequel ledit transfert indirect est réalisé en fixant lesdits segments discrets découpés de matière élastomère instable à la chaleur en des endroits isolés prédéterminés le long d'une bande continue comprenant un élément de ladite bande d'articles interconnectés.

10. Un procédé selon l'une quelconque des revendications 1-9, dans lequel ladite bande élastomère est amenée à se refroidir à la température ambiante après étirage latéral sans contrainte.

11. Un procédé selon l'une quelconque des revendications 1-10 dans lequel l'étape de découpage des articles interconnectés à partir de ladite bande intervient après l'étape de chauffage qui amène ladite matière élastomère à se contracter dans une direction à peu près parallèle à sa longueur totale.

12. Un procédé selon l'une quelconque des revendications 1-11 dans lequel les articles essentiellement non élastiques sont constitués de couches à jeter après usage comprenant une feuille support, un élément absorbant et une feuille supérieure, et dans lequel l'élément élastiquement contractable est fixé à au moins une partie de la bande de ceinture de chacune desdites couches.

**0 119 827**

Fig. 1

Fig. 2

1

## Fig. 3

## Fig. 4

## Fig. 5

Fig. 6

Fig. 7

Fig. 8

Fig. 9

Fig. 10

Fig. 11

4

Fig. 12

Fig. 13